# EUROPÄISCHE PATENTSCHRIFT

(11) **EP 0 999 828 B1**
(45) Veröffentlichungstag und Bekanntmachung des Hinweises auf die Patenterteilung: **24.09.2003**
(21) Anmeldenummer: 98943632.4
(22) Anmeldetag: 01.07.1998
(51) Int. Cl.: A61K 9/16, A61K 9/22

(54) **VERFAHREN ZUR HERSTELLUNG VON WIRKSTOFF-ZUBEREITUNGEN MIT KONTROLLIERTER FREISETZUNG AUS EINER MATRIX**
METHOD FOR PRODUCING ACTIVE AGENT PREPARATIONS WITH CONTROLLED RELEASE FROM A MATRIX
PROCEDE DE PRODUCTION DE PREPARATIONS DE PRINCIPE ACTIF AVEC LIBERATION CONTROLEE A PARTIR D'UNE MATRICE

(30) Priorität: 10.07.1997 DE 19729487
(43) Veröffentlichungstag der Anmeldung: 17.05.2000
(73) Patentinhaber: AWD.pharma GmbH & Co.KG, 01097 Dresden (DE)
(72) Erfinder: HOFFMANN, Torsten, D-01445 Radebeul (DE); LIEBOLD, Klaus, D-01307 Dresden (DE); WOLF, Joachim, D-01445 Radebeul (DE); SCHUMACHER, Heiko, D-63741 Aschaffenburg (DE)
(86) Internationale Anmeldenummer: DE9801811
(87) Internationale Veröffentlichungsnummer: WO99002134

(56) Entgegenhaltungen:
- EP-A- 0 351 580
- EP-A- 0 654 263
- WO-A-93/07859
- WO-A-93/18753
- DE-A- 1 617 593
- US-A- 3 080 293

## Beschreibung

Die vorliegende Erfindung betriftL ein neues Verfahren zur Herstellung von Zubereitungen mit kontrollierter Freisetzung, bei dem der Wirkscoff aus einer Matrix freigesetzt. wird.

Arzneiformen mit kontrollierter Freisetzung sind den therapeutischen Erfordernissen weitgehend angepaßte Darreichungsformen, deren Arzneistofftreisetzung durch Mechanismen gesteuert wird, die von physiologischen Bedingungen (pH- Wert, Enzyme, Art und Qualität der Nahrung) nicht oder in nicht signifikantem Ausmaß beeinflußt werden. Nach den angewandten Steuerungsprinzipien unterscheidet man zwischen diffusions-, matrix-, quellungs-, membran-, oder chemischkontrollierter Freisetzung. Nach der umfassenderen Definition der Amerikanischen Food and Drug Administration werden unter controlled release products Formulierungen verstanden, die bestimmt sind, den aktiven Bestandteil in Raten freizusetzen, die sich signifikant von den entsprechenden Zubereitungen mit sofortiger Freisetzung unterscheiden. Diese Definition schließt alle Typen von Retard (Depot)-Arzneiformen sowie solche mit zeitlich fixierter Freisetzung, beispielsweise magensaftresistente Präparate ein.

Aus der vorliegenden Literatur sind eine Vielzahl von Verfahren zur Herstellung derartiger Formulierungen bekannt.

Beispielsweise wird im EP 324 989 die Herstellung einer neuen galenischen Formulierung mit kontrollierter Freisetzung mittels Naßgranulation beschrieben, wobei der Wirkstoff mit entsprechenden Hilfsstoffen gemischt und in 95 %-igem Ethanol granuliert wird. Nach anschließender Trocknung wird das erhaltene Granulat auf die gewünschte Größe gesiebt.

Formulierungen mit kontrollierter Freisetzung konnen auch auf dem Wege der Schmelzgranulation hergestellt werden, wie beispielsweise in DE 24 26 812 beschrieben. Hierbei liegt der bindende Bestandteil in einem flüssigen Aggregatzustand vor, da die Prozeßtemperatur bei der Granulation höher ist als die Schmelztemperatur des niedrigschmelzenden Bcstandteils.

Weitere im Stand der Technik bekannten Verfahren zur Herstellung von Formulierungen mit kontrollierter Freisetzung sind die Granulation oder Extrusion, wie beispielsweise in DE 44 08 326 beschrieben.

Die mit den bekannten Verfahren erhaltenen WirkstoffZubereitungen weisen teilweise eine unvollständige Freisetzungsverzögerung oder große Streuungen in den Einzelwerten der Wirkstofffreisetzung auf. Das kann sich besonders nachteilig für den Patienten auswirken, da die Einhaltung der gewünschten Plasmakonzentration und damit die Bioverfügbarkeit nicht gewährleistet werden kann.

EP-A-0 654 263, WO-A-93 18753, EP-A-0 351 580 und DE-A-1 617 593 offenbaren Verfahren zur Herstellung von Wirkstoff-Zubereitungen durch Schmelzgranulation. Entsprechend EP-A-0 654 263 und WO-A-93 18753 wird das Trägermaterial im Anschluß an die Schmelzgranulation im Hochgeschwindigkeitsmischer nochmals erwärmt, wobei entsprechend EP-A-0 654 263 das Trägermaterial zu Schmelzen beginnt und anschließend weiteres Trägermaterial zugegeben wird, während entsprechend WO-A-93 18753 die Temperatur so gewählt wird, daß die zugegebene wachsartige Überzugssubstanz zu schmelzen beginnt. Entsprechend EP-A-0 351 580 ist nur ein Schmelzen der niedrigerschmelzenden Komponente der beiden Fette vorgesehen.
Die in DE-A-1 617 593 beschriebene Schmelzgranulierung von Wirkstoff und schmelzbarem Trägermaterial erfolgt unter Druck durch Verpressen zu Tabletten und entbehrt deshalb eines Abkühlungs- und Siebungsschrittes.

Der vorliegenden Erfindung liegt nun die Aufgabe zugrunde, ein neues, technisch einfaches Verfahren zur Herstellung von Formulierungen mit kontrollierter Freisetzung zu entwickeln, welches reproduzierbare Wirkstofffreisetzungen ermöglicht.

Erfindungsgemäß wird die Aufgabe dadurch gelöst, daß der Wirkstoff oder eine Wirkstoff-Kombination, allein oder mit Hilfsstoffen gemischt, unter Zugabe von Bindemitteln oder Bindemittelgemischen, die entweder bei der Zugabe geschmolzen sind oder erst während dieses Prozesses durch Erwärmung in den geschmolzenen Aggregatzustand überführt werden, granuliert, nach dem Abkühlen gesiebt und das erhaltene Granulat anschlieBend in einer Wirbelschicht unter Erwärmung nachbehandelt wird.

Es wird hierbei so vorgegangen, daß der Wirkstoff, der wasserunlöslich als auch wasserlöslich sein kann, in einem Intensivmischer vorgelegt oder vorher mit Hilfsstoffen gemischt wird und unter Zugabe von Bindemitteln oder Bindemittelgemischen, die entweder bei der Zugabe geschmolzen sind oder erst während des Prozesses im Intensivmischer durch Erwärmung in den geschmolzenen AggregaLzustand überführt werden, bei Temperaturen zwischen 10 und 100°C in einem Intensivmischer granuliert wird.

Als Intensivmischer können Schnellmischer mit oder ohne beheiz- bzw. kühlbaren Mantel wie Gral Collette oder Diosna eingesetzt werden. Besonders vorteilhaft ist das erfindungsgemäße Verfahren bei Mischern, die über keine Mantelheizung verfügen.

Nach dem Abkühlen kann das so erhaltene Granulat über ein Sieb, beispielsweise der Maschenweite 3,0 mm gesiebt und in einem Wirbelschichtgranulator in der Wirbelschicht solange erwärmt werden, bis der Schmelzpunkt des eingesetzten schmelzbaren Bindemittels im Gemisch erreicht ist. Die Nachbehandlung wird im Temperaturbereich von 30 - 100 °C unter Zufuhr erwärmter Luft bis zu dem Zeitpunkt fortgeführt, wo das Wirbelbett beinahe zusammenbricht. Anschließend wird wieder gekühlt. Die therwische Nachbehandlung wird in einem Wirbelschichtgranulator oder Wuster durchgeführt.

Nach dieser thermischen Nachbehandlung kann das nochmals gesiebte Granulat mit geeigneten Tablettierhilfsstoffen gemischt, in Kapseln gefüllt oder zu Tabletten gepreßt und gegebenenfalls mit einer Filmschicht überzogen oder dragiert werden.
Überzüge können beispielsweise solche auf der Basis von Polymethacrylsäure-Derivaten oder Cellulosederivaten sein.

Die Herstellung von Formulierungen mit kontrollierter Freisetzung nach dem erfindungsgemäßen Verfahren ist insbesondere für Substanzen aus folgenden Wirkstoffklassen geeignet:

Nonsteroidale Antiinflammatorika, Broncholytika, Vasodilatatoren, Muskelrelaxantia, Antirheumatika wie Diclotenac, Antiphlogistika, Antiepileptika wie Carbamazepin, Antihypertonika, Antihistaminika, Antikoagulantia, Darmtherapeutika, Zytostatika, Calciumantagonisten wie Verapamil und Kardiaka.

Als Bindemittel können Substanzen verwendet werden, die einen Schmelzpunkt zwischen 35 und 90 °C aufweisen. Geeignete Bindemittel sind: Wasserlösliche beziehungsweise quellbare Bindemittel wie Macrogol, Polyvidon, Polymethacrylsäure-Derivate (Eudragit), lipophile Bindemittel wie Paraffin, Cetylpalmiat, Fettalkohole, wie Cetylalkohol, Bienenwachs, Carnaubawachs, hydrierte Pflanzenöle, Triglyceride und Stearinsäure.
Diese Stoffe stellen nur eine beispielhafte Aufzählung dar, so daß auch Formulierungen mit kontrollierter Freisetzung mit anderen aktiven Substanzen und Bindemitteln auf diesem Wege hergestellt werden können.

Es war nicht vorhersehbar, daß die Formulierungen aus Granulaten, die nach dem erfindungsgemäßen Verfahren hergestellt wurden, auch bei veränderten Rezepturen reproduzierbare kontrollierte Freisetzungsraten mit sehr engen Einzelwertstreuungen aufweisen.
Durch die erfindungsgemäße thermische Nachbehandlung kann überraschenderweise eine Einstellung der spezifischen Freisetzungscharakteristiken erfolgen.

Durch diese Nachbehandlung, die durch Erwärmung in der Wirbelschicht nach vorheriger Oberflachenvergrößerung durch die Siebung erfolgt, wird eine, abhängig von den Rezepturanteilen, vollständige Einbettung beziehungsweise Umhüllung des Wirkstoffes im oder durch das Bindemittel in der Matrix erreicht. Das führt zu einer spezifikationsgerechten Freisetzungsverzögerung ohne daß unproportionale Verluste in der Wirbelschicht Abweichungen beim Wirkstoffgehalt zum Ergebnis haben.

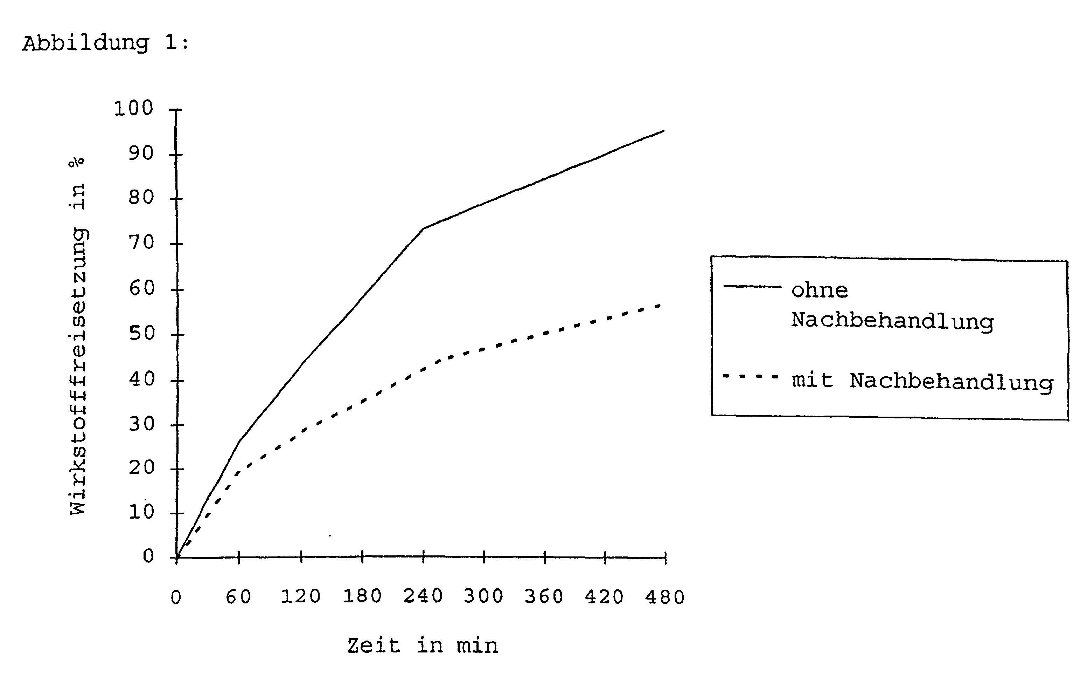

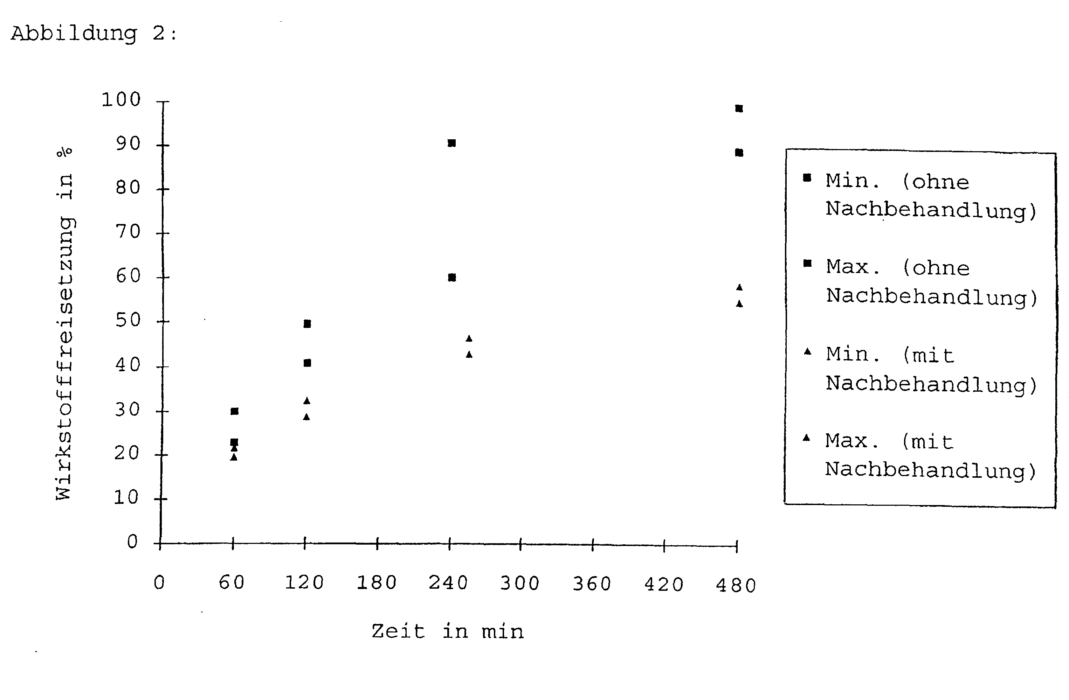

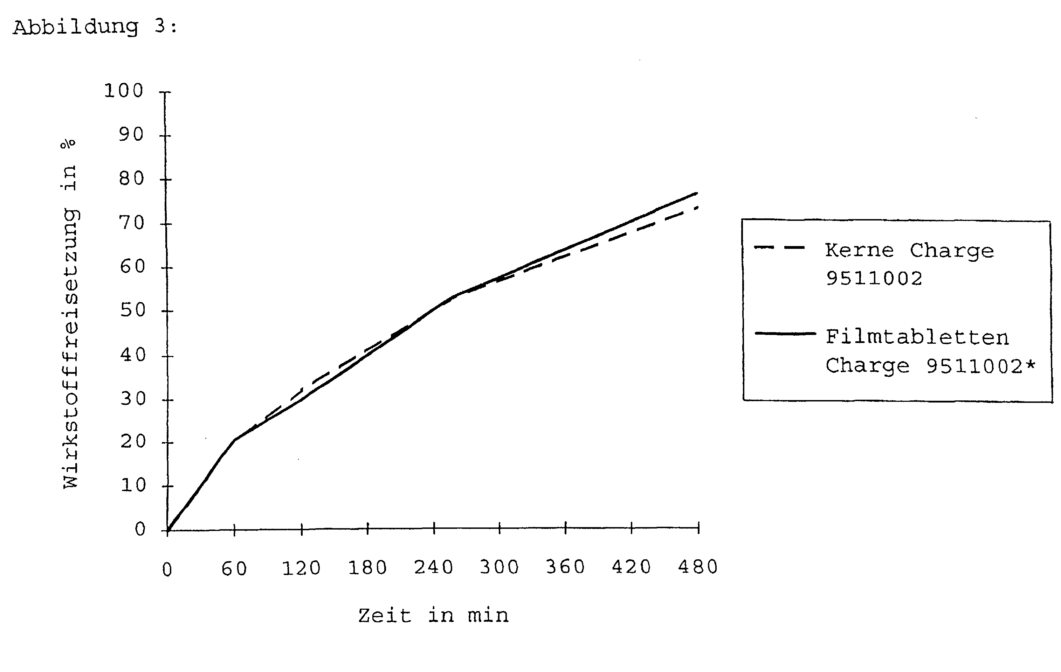

In den Abbildungen 1 - 3 wird dieser Sachverhalt verdeutlicht:
- Abbildung 1:: Auswirkung der erfindungsgemäßen Nachbehandlung auf die Wirkstofffreisetzung von Diclofenac- Natrium im Vergleich zu einem nach konventioneller Schmelzgranulation im Intensivmischer gemäß Stand der Technik ohne Nachbehandlung hergestellten Granulat
- Abbildung 2:: Vergleich der Einzelwertsbreuungen der Wirkstofffreisetzung der Darreichungsformen der nach den beiden in Abbildung 1 betrachteten Verfahren
- Abbildung 3:: Freisetzungsverzögerung, die ausschließlich durch den Kern und damit durch die Granulationsbedingungen determiniert ist.
Der Überzug hat auf die Wirkstofffreisetzung keinen signifikanten Einfluß.

Das erfindungsgemäße Verfahren ist generell anwendbar für die Realisierung spezifischer Freisetzungsraten, da durch die sichergestellte optimale Einbettung bzw. Umhüllung des oder der Arzneistoffe spezifische Verfügbarkeiten durch die Wahl eines geeigneten Bindemittels verfahrensseitig erreicht werden können.

Die nachfolgenden Beispiele sollen das erfindungsgemäße Verfahren verdeutlichen.

### Beispiel 1:

Tablette mit einem Gehalt von 100 mg Diclofenac-Natrium in mg/Tablette bestehend aus:

| | |
|---|---|
| Saccharose | 105,00 |
| Diclofenac- Natrium | 100,00 |
| Cetylalkohol | 55,20 |
| Siliciumdioxid | 0,52 |
| Magnesiumstearat | 1,30 |
| Polyvidon | 1,28 |
| Gesamt | 263,30 |

Es werden Saccharose und der Wirkstoff Diclofenac- Natrium gemischt. Anschließend erfolgt die Zugabe des geschmolzenen Cetylalkohols (Temperatur 65 ± 2°C) in einem Intensivmischer. Nach einer Granulierzeit von weniger als 10 Minuten kommt es zu einem Leistungsanstieg und die Granulation wird abgebrochen. Das Granulat wird über ein geeignetes Sieb gegeben. Danach erfolgt die Nachbehandlung in einem wirbelschichtgranulator, wobei die Zulufttemperatur 60 - 75 °C beträgt. Bei ca. 43 °C Produkttemperatur beginnt das Bindemittel zu schmelzen und die weitere Granulatbildung setzt ein. Bevor das Wirbelbett zusammenbricht ist der Endpunkt der Granulation erreicht und es wird gekühlt.

Anschließend wird nochmals gesiebt. Nach Zugabe von Siliciumdioxid, Magnesiumstearat und Polyvidon wird gemischt und die entstandene Tablettiermischung zu Tabletten mit einem Wirkstoffgehalt von 100 mg verpreßt.

### Beispiel 2:

Tablette mit einem Gehalt an 120 mg Verapamilhydrochlorid in mg/Tablette, bestehend aus:

| | |
|---|---|
| Verapamilhydrochlorid | 120 |
| Cetylalkohol | 183 |
| Cellulose | 101 |
| Siliciumdioxid | 2 |
| Magnesiumstearat | 4 |
| Gesamt | 410 |

Zum Wirkstoff Verapamilhydrochlorid wird unter Rühren in einem Intensivmischer geschmolzener Cetylalkohol gegeben. Nach kurzer Grannulierzeit wird das Granulat entnommen, gesiebt und dann in der Wirbelschicht aufgeheizt. Ab einer Produkttemperatur von ca. 43 °C setzt der gewünschte Schmelzprozeß ein, welcher bis zu einem Endpunkt bevor das Wirbelbett zusammenbricht fortgesetzt wird. Danach wird sofort gekühlt, gesiebt und mit Siliciumdioxid und Magnesiumstearat gemischt. Es werden Tabletten mit einem Gehalt von 120 mg Verapamilhydrochlorid hergestellt.

### Beispiel 3:

| | |
|---|---|
| Verapamilhydrochlorid | 120 |
| Cetylalkohol | 66 |
| Cellulose | 101 |
| Saccharose | 117 |
| Siliciumdioxid | 2 |
| Magnesiumstearat | 4 |
| Gesamt | 410 |

Die Herstellung erfolgt analog den Beispielen 1 und 2.

## Patentansprüche

1. Verfahren zur Herstellung von Wirkstoff-Zubereitungen mit kontrollierter Freisetzung aus einer Matrix **dadurch gekennzeichnet, daß** der Wirkstoff oder eine WirkstoffKombinationen, allein oder mit Hilfsstoffen gemischt, unter Zugabe von Bindemitteln oder Bindemittelgemischen, die entweder bei der Zugabe geschmolzen sind oder erst während dieses Prozesses durch Erwärmung in den geschmolzenen Aggregatzustand überführt werden, granuliert, nach dem Abkühlen gesiebt und das erhaltene Granulat anschließend in einer Wirbelschicht bei einer Mindesttemperatur, die größer gleich der Schmelztemperatur des oder der eingesetzten schmelzbaren Bindemittel ist, nachbehandelt wird.

2. Verfahren zur Herstellung nach Anspruch 1 **dadurch gekennzeichnet, daß** sowohl wasserlösliche als auch wasserunlösliche Wirkstoffe eingesetzt werden können.

3. Verfahren nach den Ansprüchen 1 und 2 **dadurch gekennzeichnet, daß** Substanzen aus folgenden Wirkstoffklassen eingesetzt werden können: nonsteroidale Antiinflammatorika, Broncholytika, Vasodilatatoren, Muskelrelaxantia, Antirheumatika, Antiphlogistika, Antiepileptika, Antihypertonika, Antihistaminika, Antikoagulantia, Darmtherapeutika, Zytostatika, Calciumantagonisten, Kardiaka.

4. Verfahren nach Anspruch 3 **dadurch gekennzeichnet, daß** als Wirkstoff Diclofenac, Verapamil oder Carbamazepin eingesetzt wird.

5. Verfahren nach Anspruch 1 **dadurch gekennzeichnet, daß** die eingesetzten Bindemittel einen Schmelzpunkt im Temperaturbereich zwischen 35°C und 90°C aufweisen.

6. Verfahren nach den Ansprüchen 1 und 5 **dadurch gekennzeichnet, daß** als Bindemittel wasserlösliche beziehungsweise quellbare Stoffe, wie Macrogol, Polyvidon, Polymethacrylsäure-Derivate oder lipophile Stoffe, wie Paraffin, Cetylpalmitat, Fettalkohole, Bienenwachs, Carnaubawachs, hydrierte Pflanzenöle, Triglyceride oder Stearinsäure verwendet werden.

7. Verfahren nach Anspruch 1 **dadurch gekennzeichnet, daß** die Granulierung in einem Temperaturbereich zwischen 10 und 100 °C durchgeführt wird.

8. Verfahren nach Anspruch 1 **dadurch gekennzeichnet, daß** die Nachbehandlung in einem Temperaturbereich zwischen 30 und 100 °C durchgeführt wird.

9. Verfahren nach Anspruch 1 **dadurch gekennzeichnet, daß** als Granulator ein Intensivmischer mit oder ohne beheizbeziehungsweise kühlbaren Mantel eingesetzt wird.

10. Verfahren nach Anspruch 1 **dadurch gekennzeichnet, daß** die thermische Nachbehandlung in einem Wirbelschichtgranulator durchgeführt wird.

11. Pharmazeutische Zubereitungen, enthaltend Wirkstoff-Granulat, hergestellt gemäß Anspruch 1 **dadurch gekennzeichnet, daß** das so hergestellte Granulat in Kapseln gefüllt oder zu Tabletten verpreßt und gegebenenfalls mit einer Filmschicht überzogen oder dragiert wird.

12. Wirkstoff-Zubereitungen, hergestellt gemäß Anspruch 1.

## Claims

1. Process for the production of active ingredient compositions with controlled release from a matrix, **characterized in that** the active ingredient or a combination of active ingredients is granulated, alone or mixed with ancillary substances, with the addition of binders or mixtures of binders, which are either molten on addition or first converted into the molten state of aggregation by heating during this process, and are screened after the cooling, and the resulting granules subsequently undergo after-treatment in a fluidized bed at a minimum temperature which is greater than or equal to the melting point of the fusible binder(s) employed.

2. Process for production according to Claim 1, **characterized in that** it is possible to employ both water-soluble and water-insoluble active ingredients.

3. Process according to Claims 1 and 2, **characterized in that** substances from the following classes of active ingredients can be employed: non-steroidal antiinflammatory drugs, bronchodilators, vasodilators, muscle relaxants, antirheumatics, antiinflammatory agents, antiepileptics, antihypertensives, antihistamines, anticoagulants, intestinal therapeutic agents, cytostatics, calcium antagonists, cardiac remedies.

4. Process according to Claim 3, **characterized in that** diclofenac, verapamil or carbamazepine is employed as active ingredient.

5. Process according to Claim 1, **characterized in that** the binders employed have a melting point in the temperature range between 35°C and 90°C.

6. Process according to Claims 1 and 5, **characterized in that** the binders used are water-soluble or swellable substances such as macrogol, polyvidone, polymethacrylic acid derivatives or lipophilic substances such as paraffin, cetyl palmitate, fatty alcohols, beeswax, carnauba wax, hydrogenated vegetable oils, triglycerides or stearic acid.

7. Process according to Claim 1, **characterized in that** the granulation is carried out in the temperature range between 10 and 100°C.

8. Process according to Claim 1, **characterized in that** the after-treatment is carried out in a temperature range between 30 and 100°C.

9. Process according to Claim 1, **characterized in that** an intensive mixer with or without heatable or coolable jacket is employed as granulator.

10. Process according to Claim 1, **characterized in that** the thermal after-treatment is carried out in a fluidized bed granulator.

11. Pharmaceutical compositions containing active ingredient granules produced according to Claim 1, **characterized in that** the granules produced in this way are packed into capsules or compressed to tablets and, where appropriate, coated with a film layer or coated with sugar.

12. Active ingredient compositions produced according to Claim 1.

## Revendications

1. Procédé de production de préparations de principe actif ayant une libération contrôlée à partir d'une matrice,
**caractérisée en ce qu'**
on convertit le principe actif ou la combinaison de principes actifs, isolément ou en mélange avec des substances auxiliaires, par addition d'agent liant ou de mélanges d'agents liants qui sont fondus soit lors de l'addition ou, avant cela, pendant ce processus, par chauffage à l'état d'agrégats fondus, on granule, ou tamise après refroidissement et on retraite le granulé obtenu ensuite en couche tourbillonnaire à une température minimale qui est supérieure ou égale à la température de fusion du ou des agent(s) liant(s) fusible(s) mis en oeuvre.

2. Procédé de production selon la revendication 1,
**caractérisé en ce qu'**
un peut mettre en oeuvre des principes actifs aussi bien soluble dans l'eau qu'insolubles dans l'eau.

3. Procédé selon les revendications 1 et 2,
**caractérisé en ce qu'**
on peut mettre en oeuvre des substances choisies dans les classes de principes actifs suivants : anti-inflammatoires non stéroïdiens, broncholytiques, vasodilatateurs, relaxants musculaires, antirhumatismaux, antiphlogistiques, antiépileptiques, antihypertoniques, antihistaminiques, anticoagulants, agents thérapeutiques intestinaux, cytostatiques. antagonistes du calcium, produits cardiaques.

4. Procédé selon la revendication 3,
**caractérisé en ce qu'**
on met en oeuvre comme principe actif du diclofénac, du Vérapamil ou de la carbamazépine.

5. Procédé selon la revendication 1,
**caractérisé en ce que**
les agents liants mis en oeuvre possèdent un point de fusion dans la plage de températures comprise entre 35°C et 90°C.

6. Procédé selon les revendications 1 et 5.
**caractérisé en ce qu'**
on utilise des substances solubles dans l'eau, ou qui peuvent gonfler dans l'eau comme le macrogol, la polyvidone les dérivés de l'acide polyméthacrylique, ou des substances lipophiles comme la paraffine, le palmitate de cétyle, des alcools gras, la cire d'abeilles, la cire de carnauba, des huiles végétales hydrogénées, des triglycérides ou de l'acide stéarique.

7. Procédé selon la revendication 1.
**caractérisé en ce qu'**
on effectue la granulation dans une plage de températures comprise entre 10 et 100°C.

8. Procédé selon la revendication 1,
**caractérisé en ce qu'**
on effectue le retraitement dans une plage de températures comprise entre 30 et 100°C.

9. Procédé selon la revendication 1,
**caractérisé en ce qu'**
on met en oeuvre comme granulateur un mélangeur intensif avec ou sans enveloppe, qu'on peut chauffer ou refroidir.

10. Procédé selon la revendication 1,
**caractérisé en ce qu'**
on effectue le retraitement thermique dans un granulateur à lit tourbillonaire.

11. Préparations pharmaceutiques contenant un granulé de principe actif, produit conformément à la revendication 1,
**caractérisées en ce que**
le granulé ainsi produit est réparti en capsules ou pressé en comprimés et le cas échéant revêtu d'une couche de film ou dragéifié.

12. Préparations de principe actif produits conformément à la revendication 1.
